(19) Europäisches Patentamt
European Patent Office
Office européen des brevets

(11) **EP 3 296 739 A1**

(12) **EUROPÄISCHE PATENTANMELDUNG**

(43) Veröffentlichungstag:
**21.03.2018   Patentblatt 2018/12**

(51) Int Cl.:
***G01N 33/49*** *(2006.01)*    ***G01N 21/31*** *(2006.01)*

(21) Anmeldenummer: **16188802.9**

(22) Anmeldetag: **14.09.2016**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Siemens Healthcare Diagnostics Products GmbH**
**35041 Marburg (DE)**

(72) Erfinder:
• **Patzke, Juergen**
**35043 Marburg (DE)**
• **Zander, Norbert**
**35039 Marburg (DE)**

(54) **VERFAHREN UND SYSTEM ZUR ZUORDNUNG EINER KÖRPERFLÜSSIGKEITSPROBE ZU EINER KÖRPERFLÜSSIGKEITSPROBENKLASSE**

(57)    Die Erfindung betrifft ein Verfahren zur Zuordnung einer Körperflüssigkeitsprobe zu einer Körperflüssigkeitsprobenklasse aus der Gruppe Citrat-Plasma (PAP), Citrat-Plasma mit Bilirubin (B) oder Hämoglobin (H), Serum mit Bilirubin oder Hämoglobin, lipämisches Plasma (T) oder Serum, EDTA-Plasma (E), Serum (S), und plättchenreiches Plasma (P), und umfasst das Durchstrahlen der Körperflüssigkeitsprobe mit Licht bei einer Mehrzahl von Wellenlängen und Messung einer Mehrzahl von Absorptionen (A1, A2) der Körperflüssigkeitsprobe bei der Mehrzahl von Wellenlängen.

FIG 1

EP 3 296 739 A1

**Beschreibung**

Die vorliegende Erfindung bezieht sich auf ein Verfahren und ein System zur Zuordnung einer Körperflüssigkeitsprobe zu einer Körperflüssigkeitsprobenklasse, insbesondere einer Körperflüssigkeitsprobenklasse aus der Gruppe Citrat-Plasma, lipämisches Plasma oder Serum, EDTA-Plasma, Serum, Plasma oder Serum mit Bilirubin, Plasma oder Serum mit Hämoglobin, und plättchenreiches Plasma.

Zahlreiche Nachweis- und Analyseverfahren zur Bestimmung physiologischer Parameter in Körperflüssigkeitsproben beruhen auf optischen, insbesondere photometrischen, Messprinzipien. Optische und insbesondere photometrische Verfahren ermöglichen den qualitativen und quantitativen Nachweis von Analyten in flüssigen Proben.

Die Bestimmung klinisch relevanter Parameter, wie zum Beispiel der Konzentration oder der Aktivität eines Analyten erfolgt vielfach, indem ein Aliquot einer Körperflüssigkeit eines Patienten mit einem oder mehreren Testreagenzien in vitro vermischt wird, wodurch eine Reaktion in Gang gesetzt wird, die eine messbare Veränderung einer optischen Eigenschaft des Testansatzes bewirkt. Eine solche Veränderung könnte zum Beispiel jeweils eine Zu- oder Abnahme der Absorption, z.B. Turbidimetrie, der Lichtstreuung, z.B. Nephelometrie, der Intensität der messbaren Fluoreszenz oder der Intensität der messbaren Chemilumineszenz sen. Die Photometrie untersucht und nutzt die Schwächung oder die Verstärkung eines Lichtstroms beim Durchtritt durch ein absorbierendes und/oder streuendes Medium. Je nach Art der ausgelösten Reaktion kommen unterschiedliche photometrische Messverfahren zum Einsatz, die die Messung eines trüben flüssigen Testansatzes ermöglichen.

Hierzu können turbidimetrische Verfahren eingesetzt werden, bei denen die Trübung beziehungsweise die optische Dichte einer Lösung oder Suspension anhand der Lichtschwächung oder Verstärkung des Lichtdurchgangs eines direkt durch die Suspension hindurch tretenden Lichtstrahls gemessen wird.

Die Intensität des Lichtstrahls nimmt beim Durchtritt durch eine Messzelle beziehungsweise Küvette, die eine flüssige Probe enthält, ab. Die Verluste können durch Interaktionen des Lichtstrahls mit der in der Messzelle befindlichen Probe, beispielsweise durch Absorptions-, Diffraktions-, Streuungs- und/oder Reflexionseffekte beeinflusst werden. Im Allgemeinen können Diffraktions-, Beugungs- und Reflexionseffekte vernachlässigt beziehungsweise durch Referenzmessungen ausgeglichen werden, so dass hauptsächlich die Absorption zur Schwächung des Lichtstrahls beiträgt. Bei Testen zur Bestimmung von Analyten kann die zu messende Reaktion entweder die Absorption verstärken oder verringern.

Photometrische Konzentrationsbestimmungen beruhen daher auf einer gesetzmäßigen Abhängigkeit der Extinktion beziehungsweise Absorption von der Konzentration der gelösten Stoffe und der Schichtdicke der Messzelle bei einer bestimmten Wellenlänge des eingestrahlten Lichts. Diesen Zusammenhang beschreibt das Lambert-Beer-Bouguer-Gesetz:

$$E(\lambda) = -\log(I/I_0) = \varepsilon(\lambda) \cdot c \cdot d \qquad\qquad (1)$$

wobei $E(\lambda)$ die von der Wellenlänge $\lambda$ des Lichtstrahls abhängige Extinktion, I die Lichtintensität nach Durchtritt durch die Probe, $I_0$ die Lichtintensität vor Durchtritt durch die Probe, $\varepsilon(\lambda)$ der wellenlängenabhängige molare Extinktionskoeffizient eines durchstrahlten Stoffes, c die molare Konzentration des durchstrahlten Stoffes und d die durch den Lichtstrahl durchstrahlte Schichtdicke, beispielsweise der Messzelle ist.

Anhand der Extinktion $E(\lambda)$ einer Probe lässt sich die Konzentration einer Substanz in einer Lösung ermitteln. Dazu ist es erforderlich, dass zuvor die Extinktion mindestens einer Standardlösung bekannter Konzentration bestimmt wurde. Da sich die Extinktion proportional zur Konzentration verhält, kann mittels Kalibration durch Extinktionsmessungen mehrerer Standardlösungen bekannter Konzentrationen die Konzentration einer gelösten Substanz ermittelt werden.

Die Extinktion einer Probe hängt jedoch nicht nur von der Konzentration der zu bestimmenden Substanz selbst ab, sondern auch von der Art der Probenmatrix. Die Extinktionen verschiedener Substanzen verhalten sich in einem Gemisch additiv, sofern die Substanzen nicht untereinander wechselwirken. Körperflüssigkeiten, wie beispielsweise Blutplasma oder Blutserum sind jeweils komplexe Gemische und enthalten neben dem zu bestimmenden Analyten eine Vielzahl weiterer Substanzen, die die Gesamtabsorption der Probe beeinflussen.

Körperflüssigkeitsproben können in Einzelfällen auch abnormal hohe Konzentrationen einer oder mehrerer endogener, also körpereigener Substanzen enthalten, die sich bei Überschreitung einer tolerablen Konzentration in optischen, insbesondere photometrischen, Detektionsverfahren als störend erweisen und sich zu einem systematischen Fehler auswirken können. Auch exogene Substanzen wie zum Beispiel Fettemulsionen, die beispielsweise bei künstlicher Ernährung eingesetzt werden, können stören.

Probleme bereiten bekanntermaßen hämolytische, ikterische und/oder lipämische Serum- oder Plasmaproben, die über abnormal hohe Hämoglobin-, Bilirubin- und/oder Lipid-Konzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden. Werden solche Proben einem photometrischen Verfahren unterworfen, das der Bestimmung eines analytischen, diagnostisch relevanten Parameters dient, besteht die Gefahr einer Fehlbestimmung die gegebenenfalls eine Fehldiagnose und schlimmstenfalls eine Fehlbehandlung des Patienten zur Folge haben kann. Die präanalytische Identifikation hämolytischer, ikterischer sowie lipämischer Proben ist also zur Vermeidung von fehlerhaften Analyseergebnissen von besonderer Wichtigkeit. Ähnliches gilt für Proben mit erhöhtem Thrombozytengehalt.

Darüber hinaus können Hämoglobin-, Bilirubin-, Triglyzeridund Pättchengehalte diagnostische Tests auch über nichtoptische, wie zum Beispiel biochemische, Mechanismen stören.

Weiter bereitet das Verwechseln von Proben, die unterschiedlichen Probentypen angehören, häufig Probleme. Zu den besonders häufig miteinander verwechselten Probentypen gehören Citrat-Plasma, EDTA-Plasma, Serum sowie plättchenreiches Plasma. Fehlerhafte Ergebnisse von Labortests und darauf fußende Fehldiagnosen mit teilweise sehr nachteiligen und weitreichenden Folgen für den Gesundheitszustand und die Lebenserwartung der betroffenen Patienten lassen sich relativ häufig auf das Verwechseln von Proben verschiedener Körperflüssigkeitsprobenklassen zurückführen.

Es besteht daher ein dringender Bedarf an Verfahren zur Zuordnung einer Körperflüssigkeitsprobe zu bestimmten Probenklassen von Körperflüssigkeitsproben.

In der EP 2549264 A1 ist ein Verfahren zur Bestimmung von Bilirubin, Hämoglobin und Lipiden in Plasma- oder Serumproben beschrieben.

Die bisher bekannten Verfahren sind nicht optimal bezüglich der Differenzierung von Lipämie, Bilirubin oder Hämoglobin-Interferenzen. Eine Verwechslung des Probentyps, beispielsweise EDTA-Plasma, Serum oder plättchenreiches Plasma, wird nicht erkannt. Von besonderer praktischer Bedeutung ist das Erkennen von plättchenreichen PlasmaProben und deren Unterscheidung von Citrat-Plasma, Citrat-Plasma mit Bilirubin oder Hämoglobin, lipämischem Plasma oder Serum und/oder EDTA-Plasma.

Der vorliegenden Erfindung liegt demnach die Aufgabe zugrunde, ein Verfahren zur spektrophotometrischen Zuordnung einer Körperflüssigkeitsprobe zu bestimmten Probentypen von Körperflüssigkeitsproben zur Verfügung zu stellen.

**Diese Aufgabe wird durch das erfindungsgemäße Verfahren gelöst.**

Eine Ausführungsform der vorliegenden Erfindung besteht in einem Verfahren zur Zuordnung einer Körperflüssigkeitsprobe zu einer Körperflüssigkeitsprobenklasse aus der Gruppe Citrat-Plasma, Plasma oder Serum mit Bilirubin, Plasma oder Serum mit Hämoglobin, lipämisches Plasma oder Serum, EDTA-Plasma, Serum, und plättchenreiches Plasma, mit den Schritten:

a) Durchstrahlen der Körperflüssigkeitsprobe mit Licht bei zwei Wellenlängen und
b) Messung der Absorptionen der Körperflüssigkeitsprobe bei den zwei Wellenlängen;

gekennzeichnet durch die Schritte:

c) Messung einer ersten Absorption A1 der Körperflüssigkeitsprobe bei einer ersten Wellenlänge, die im Bereich zwischen 610 nm und 930 nm liegt,
d) Messung einer zweiten Absorption A2 der Körperflüssigkeitsprobe bei einer zweiten Wellenlänge, die im Bereich zwischen 320 nm und 420 nm liegt,
e) Vergleich der ersten Absorption A1 mit einem vorbestimmten ersten Grenzwert G1 der Absorption,
f) Bildung eines von der ersten Absorption A1 abhängigen zweiten Grenzwerts G2 der Absorption und Vergleich der zweiten Absorption A2 mit dem zweiten Grenzwert G2 der Absorption,
g) Zuordnung der Körperflüssigkeitsprobe zu der Körperflüssigkeitsprobenklasse plättchenreiches Plasma, falls A1 größer G1 und A2 kleiner G2, oder
h) Zuordnung der Körperflüssigkeitsprobe zu der Körperflüssigkeitsprobenklasse Citrat -Plasma, Plasma mit Bilirubin oder Hämoglobin, lipämisches Plasma oder Serum, Serum oder EDTA-Plasma, falls A1 kleiner G1 oder A2 größer G2,

wobei Citrat-Plasma, Plasma oder Serum mit Bilirubin, Plasma oder Serum mit Hämoglobin, lipämisches Plasma oder Serum, Serum, oder EDTA-Plasma eine Körperflüssigkeitsprobenklasse bildet und wobei plättchenreiches Plasma eine Körperflüssigkeitsprobenklasse bildet.

Somit können mittels des erfindungsgemäßen Verfahrens Körperflüssigkeitsproben der Körperflüssigkeitsprobenklasse plättchenreiches Plasma von Körperflüssigkeitsproben der Körperflüssigkeitsprobenklasse Citrat-Plasma, Plasma oder Serum mit Bilirubin, Plasma oder Serum mit Hämoglobin, lipämisches Plasma oder Serum, Serum und/oder EDTA-Plasma unterschieden werden.

Gemäß einer vorteilhaften Ausführungsform des Verfahrens beträgt die erste Wellenlänge 850 nm und/oder die zweite Wellenlänge 340 nm.

Gemäß einer weiteren vorteilhaften Ausführungsform des Verfahrens beträgt die erste Wellenlänge 645 nm und/oder die zweite Wellenlänge 365 nm.

Gemäß einer weiteren vorteilhaften Ausführungsform des Verfahrens beträgt der erste Grenzwert der Absorption G1 0,15 A und/oder der zweite Grenzwert der Absorption G2 0,7189 ln(A1) + 2,3413 A.

Die Absorption kann in der Einheit A angegeben werden und bezieht sich hier auf Messungen an eine Küvette der Schichtdicke 10 mm. Die Absorption der Küvette selbst (Referenzwert) wurde nicht abgezogen. In Abhängigkeit des verwendeten Photometers und der Schichtdicke der Küvette sowie den Absorptionseigenschaften der Küvette sind die Grenzwerte der Absorption entsprechend anzupassen und zu optimieren.

Die Bestimmung eines Grenzwertes der Absorption kann beispielsweise durch eine selektive Festlegung des Grenzwertes erfolgen. Eine vergleichsweise einfache Festlegung kann so funktionieren, dass bestimmte Körperflüssigkeitsprobenklassen ausgewählt werden, deren Absorption sich erwartungsgemäß mittels des Grenzwerts unterscheiden lassen. Beispielsweise wird der erste Grenzwert G1 der Absorption mittels eines Vergleichs der Absorption bei der ersten Wellenlänge, beispielsweise bei 850 nm, zwischen Proben der Klassen Plasma, Serum und EDTA-Plasma einerseits und Proben der Klassen plättchenreiches Plasma und lipämisches Plasma andererseits, bestimmt, da erwartet wird, dass sich die Proben der Klassen Plasma, Serum und EDTA-Plasma durch eine niedrigere Extinktion bei 850 nm von den Proben der Klassen plättchenreiches Plasma und lipämisches Plasma unterscheiden. Benötigt wird eine statistisch ausreichende Zahl eindeutig charakterisierter Proben dieser Klassen. Plasma, Serum und EDTA-Plasma werden in einer Gruppe zusammengefasst. Für diese Gruppe wird ein Normalbereich bestimmt, so dass z.B. die 95% Perzentile der Extinktion bei 850 nm den Grenzwert ergibt. Alternativ kann der Normalbereich auch für die Gruppe mit den beiden Klassen plättchenreiches Plasma und lipämisches Plasma bestimmt werden und dann z.B. die 5% Perzentile der Extinktion bei 850 nm verwendet werden. Eine weitere Möglichkeit ist, beide Verfahren anzuwenden und daraus den Mittelwert als Grenzwert zu verwenden. Alternativ kann auch mittels anderer mathematischer Verfahren der Normalbereich bestimmt werden, z.B. die mittels der zweifachen Standardabweichung.

Das beschriebene Vorgehen zur Bestimmung eines Grenzwertes der Absorption kann analog bei allen Bedingungen mit einem festen Grenzwert der Absorption bei einer Wellenlänge angewendet werden.

Der zweite Grenzwert der Absorption G2, beispielsweise 0,7189 ln(A1) + 2,3413 A, ist ein Beispiel für eine mathematische Formel (zur Unterscheidung von plättchenreichem Plasma einerseits und lipämischem Plasma oder Serum, Serum, Citrat-Plasma, Plasma oder Serum mit Bilirubin, Plasma oder Serum mit Hämoglobin andererseits. Als Ausgangsbasis wird eine statistisch ausreichende Zahl eindeutig charakterisierter Proben aus den Klassen plättchenreiches Plasma und lipämisches Plasma oder Serum, Serum, Citrat-Plasma, Plasma oder Serum mit Bilirubin, Plasma oder Serum mit Hämoglobin benötigt, die keine weiteren Interferenzen aufweisen. Zur Festlegung der Formel gibt es wiederum verschieden Möglichkeiten. Der Fachmann kann visuell eine logarithmische Funktion auswählen und Parameter der logarithmischen Funktion ermitteln, so dass sich Proben der Klassen lipämisches Plasma oder Serum, Serum, Citrat-Plasma, Plasma oder Serum mit Bilirubin, Plasma oder Serum mit Hämoglobin und plättchenreiches Plasma besonders eindeutig mittels des Verlaufs des Graphen der logarithmischen Funktion unterscheiden lassen.

Alternativ wird beispielsweise mittels der Absorptionswerte der plättchenreichen Plasma Proben ein Graph einer anderen Funktion (z.B. ein Polynom) angepasst. Für eine solche Funktion kann ein Vertrauensbereich mathematisch berechnet werden. Dessen Grenze differenziert beispielsweise plättchenreiches Plasma von lipämischem Plasma oder Serum, Serum, Citrat-Plasma, Plasma oder Serum mit Bilirubin, Plasma oder Serum mit Hämoglobin. Alternativ kann der Graph der Funktion auch durch die lipämischen Plasma oder Serum, Serum, Citrat-Plasma, Plasma oder Serum mit Bilirubin, Plasma oder Serum mit Hämoglobin Proben laufen und der Vertrauensbereich entsprechend berechnet werden, der diese Proben von plättchenreichem Plasma differenziert. Ebenso können auch beide Vertrauensbereiche kombiniert werden. Dies kann beispielsweise so umgesetzt werden, dass für jedes Wertepaar der Extinktion bei 340 nm und bei 850 nm jeweils ein Grenzwert berechnet wird und der Mittelwert beider Grenzwerte als finaler Grenzwert verwendet wird.

Bevorzugt kann die Bestimmung eines Grenzwertes der Absorption beispielsweise durch eine globale Festlegung erfolgen. Es wird für jede Klasse von Proben eine statistisch ausreichende Anzahl von eindeutig charakterisierten Proben benötigt. Die Extinktion der Proben bei den vom Algorithmus benötigten Wellenlängen wird gemessen. Es wird ein vorläufiger Grenzwert festgelegt, z.B. wie bezüglich der selektiven Festlegung beschrieben. Dann wird für jede Bedingung der Grenzwert iterativ optimiert. Nach jedem Iterationsschritt wird für alle Proben erneut die Klassenzuordnung berechnet. Es verändert sich also nach jedem Schritt möglicherweise der Prozentsatz richtig zugeordneter Proben bei den verschiedenen Klassen. Daher kann bei einer solchen Vorgehensweise gezielt die Anforderung eines bestimmten Labors abgebildet werden. So könnte z.B. ein Labor, bei dem überhaupt keine Serum- oder EDTA-Plasma Proben benutzt werden, eine ungünstige Sensitivität der Klassenzuordnung bei diesen Proben akzeptieren. Die Grenzwerte könnten z.B. im Hinblick auf eine besonders sensitive Erkennung von lipämischen Plasma Proben optimiert werden.

In bevorzugter Ausgestaltung des erfindungsgemäßen Verfahrens ist das Verfahren bezüglich der Charakterisierung der Klassen assayspezifisch optimiert. Beispielsweise kann das erfindungsgemäße Verfahren testspezifisch an die tatsächliche Störung des Assays durch z.B. erhöhte Plättchenzahl (PRP) angepasst werden. Dazu werden die Proben innerhalb einer Klasse "erhöhte Plättchenzahl (PRP)" weiter aufgeteilt. Solche Proben, bei denen bei dem untersuchten Test eine Störung auftritt, werden einer Klasse "erhöhte Plättchenzahl (PRP) mit Störung" zugeordnet, die besonders sensitiv erkannt werden soll. Eine Klasse "erhöhte Plättchenzahl (PRP) ohne Störung" wird separat betrachtet und muss nicht richtig erkannt werden, da keine Störung auftritt. Bei einer solchen Vorgehensweise müssen entsprechende Warnhinweise testspezifisch ausgegeben werden. So kann z.B. bei der gleichen Probe der Test X den Warnhinweis hervorrufen "Vorsicht - Interferenz erhöhte Plättchenzahl (PRP)", während beim Test Y nach Messung der gleichen Probe kein Warnhinweis erscheint. Diese Vorgehensweise ist grundsätzlich in Analogie bei allen Klassen von Körperflüssigkeitsproben und Interferenzen anwendbar.

Gemäß einer weiteren vorteilhaften Ausführungsform des Verfahrens erfolgt das Durchstrahlen der Körperflüssigkeitsprobe mithilfe von mindestens einer, bevorzugt zwei bis 10, besonders bevorzugt vier bis sechs Laser - und/oder Leuchtdioden und das Erfassen der Mehrzahl von Absorptionen (A1; A2) mithilfe mindestens eines photometrischen Sensors. Die hat den Vorteil, dass das Verfahren besonders schnell und kostengünstig durchführbar ist.

Die Erfindung betrifft weiter ein System, z.B. ein Analysegerät, zur Zuordnung einer Körperflüssigkeitsprobe zu einer Körperflüssigkeitsprobenklasse aus der Gruppe Citrat-Plasma, Plasma oder Serum mit Bilirubin, Plasma oder Serum mit Hämoglobin, lipämisches Plasma oder Serum, Serum, oder EDTA-Plasma, und plättchenreiches Plasma, mit:

I) einer Messeinrichtung, welche dazu ausgelegt ist, eine Körperflüssigkeitsprobe mit Lichtstrahlen mit zwei Wellenlängen zu durchstrahlen und Messung von zwei Absorptionen der Körperflüssigkeitsprobe bei den zwei Wellenlängen durchzuführen, wobei
die Messung einer ersten Absorption A1 der Körperflüssigkeitsprobe bei einer ersten Wellenlänge erfolgt, die im Bereich zwischen 610 nm und 930 nm liegt, und die Messung einer zweiten Absorption A2 der Körperflüssigkeitsprobe bei einer zweiten Wellenlänge erfolgt, die im Bereich zwischen 320 nm und 420 nm liegt, und

II) einer Berechnungseinrichtung, welche dazu ausgelegt ist, die erste Absorption A1 mit einem vorbestimmten ersten Grenzwert G1 der Absorption zu vergleichen, und

III) einer Berechnungseinrichtung, welche dazu ausgelegt ist, einen von der ersten Absorption A1 abhängigen zweiten Grenzwert G2 der Absorption zu bilden und die zweite Absorption A2 mit dem zweiten Grenzwert G2 der Absorption zu vergleichen, und

IV) einer Berechnungseinrichtung, welche dazu ausgelegt ist, die Körperflüssigkeitsprobe zu der Körperflüssigkeitsprobenklasse plättchenreiches Plasma zuzuordnen, falls A1 größer G1 und A2 kleiner G2 ist, oder die Körperflüssigkeitsprobe zu der Körperflüssigkeitsprobenklasse Citrat-Plasma, Plasma oder Serum mit Bilirubin, Plasma oder Serum mit Hämoglobin, lipämisches Plasma oder Serum, Serum, oder EDTA-Plasma zuzuordnen, falls A1 kleiner G1 oder A2 größer G2 ist,

wobei Citrat-Plasma, Plasma oder Serum mit Bilirubin, Plasma oder Serum mit Hämoglobin, lipämisches Plasma oder Serum, Serum, oder EDTA-Plasma eine Körperflüssigkeitsprobenklasse bildet und
wobei plättchenreiches Plasma eine Körperflüssigkeitsprobenklasse bildet.

In einer bevorzugten Ausführung des Systems beträgt
die erste Wellenlänge 850 nm und/oder die zweite Wellenlänge 340 nm.

In einer bevorzugten Ausführung des Systems beträgt
die erste Wellenlänge 645 nm und/oder die zweite Wellenlänge 365 nm.

In einer weiteren bevorzugten Ausführung des Systems beträgt der erste Grenzwert der Absorption G1 0,15 A und/oder der zweite Grenzwert der Absorption G2 0,7189 ln(A1) + 2,3413 A.

In einer bevorzugten Ausführung des Systems erfolgt das Durchstrahlen der Körperflüssigkeitsprobe mithilfe von Laser- oder Leuchtdioden und das Erfassen der Mehrzahl von Absorptionen (A1; A2) mithilfe eines photometrischen Sensors.

Vorteilhafterweise kann die Körperflüssigkeitsprobe Blutserum oder Blutplasma umfassen.

Ein weiterer Gegenstand der Erfindung ist ein automatischer Analysator umfassend ein erfindungsgemäßes System.

Ein weiterer Gegenstand der Erfindung ist die Verwendung eines erfindungsgemäßen Systems in einem automatischen Analysator.

Der Begriff "plättchenreiches Plasma" umfasst eine Plasmaprobe eines Individuums, bevorzugt eine Plasmaprobe eines menschlichen Individuums, bei der durch die schwache Zentrifugation erreicht wurde, dass die Thrombozyten nicht sedimentieren und somit in der Plasmaprobe verbleiben. Es gibt keine eindeutige Definition, welche Mindestthrombozytenzahl ein plättchenreiches Plasma enthalten muss. Man würde zum Beispiel auch bei einer sehr schweren Thrombozytopenie von < 10000 Thrombozyten / μL die Probe als plättchenreiches Plasma bezeichnen. Im Sinne dieser Erfindung, bei der ein Plasma mit einer gegenüber des plättchenarmen Plasmas deutlich erhöhten Thrombozytenzahl detektiert werden soll, wird plättchenreiches Plasma so definiert, dass die Probe wie für die Herstellung von plättchenreichem Plasma typisch zentrifugiert wurde. Dem Fachmann sind diverse Verfahren zur Gewinnung von plättchenreichem Plasma aus einer Vollblutprobe bekannt. Ein übliches Verfahren funktioniert wie folgt: Eine antikoagulierte Vollblutprobe wird bei 170 g für 15 Minuten oder 150 g für 15 Minten oder bei 180 g für 10 Minuten zentrifugiert. Dadurch wird erreicht, dass sich eine untere Schicht aus roten und weißen Blutzellen bildet und darüber eine Schicht plättchenreiches Plasma. Letzteres wird vorsichtig ohne Vermischung mit der unteren Schicht entnommen.

Plättchenarmes Plasma hingegen, das üblicherweise gemeint ist, wenn der Begriff "Plasma" verwendet wird, ist idealerweise plättchenfrei, muss aber in jedem Fall weniger als 10.000 Plättchen pro Mikroliter [μL] Probe enthalten. In der klinischen Praxis bei Anwendung typischer Zentrifugationsprozedere (siehe unten) für die Herstellung von plättchenarmen Plasma werden aber häufig noch Thrombozytenzahlen > 10.000/μL gefunden. Im Sinne dieses Verfahrens, welches eindeutig plättchenreiche Proben von plättchenarmen Proben unterscheiden soll, werden auch noch Proben, die nach Zentrifugationsverfahren für plättchenarmes Plasma hergestellt wurden, als solche definiert, wenn sie durch Zentrifugation wie für plättchenarmes Plasma typisch hergestellt wurden. Wenn das erfindungsgemässe Verfahren für die Erkennung von schwach erhöhten Thrombozytenzahlen eingesetzt werden soll, kann diese definierte Grenze auch auf bis zu 10000 / μL angesetzt werden. Dem Fachmann sind diverse Verfahren zur Gewinnung von plättchenarmem Plasma aus einer Vollblutprobe bekannt. Ein übliches Verfahren funktioniert wie folgt: Eine antikoagulierte Vollblutprobe wird bei 1500 g für mindestens 15 Minuten oder bei 1500 g für 10 Minuten oder bei 2000 g für 20 Minuten zentrifugiert. Dadurch wird erreicht, dass sich eine untere Schicht aus roten und weißen Blutzellen und aus Plättchen bildet und darüber eine Schicht plättchenarmes Plasma. Letzteres wird vorsichtig ohne Vermischung mit der unteren Schicht entnommen.

Bei plättchenarmem Plasma handelt es sich um "Citrat-Plasma". Die Begriffe "plättchenarmes Plasma", "(Citrat-)Plasma", "Plasma" sowie "Citrat-Plasma" werden synonym verwendet und bezeichnen die gleiche Körperflüssigkeitsprobenklasse.

Der Begriff "Citrat-Plasma" umfasst eine Plasmaprobe eines Individuums, bevorzugt eine Plasmaprobe eines menschlichen Individuums, von dessen Blut 9 Volumenanteile mit einem Volumenanteil Natriumzitratlösung antikoaguliert wurde. Weit verbreitet sind 3,2 %ige und 3,8 %ige Natriumzitratlösungen. Die Blutprobe wird zur Gewinnung des Plasma-Überstandes zentrifugiert.

Der Begriff "lipämisches Plasma oder Serum" umfasst eine Plasmaprobe eines Individuums, bevorzugt eine Plasmaprobe eines menschlichen Individuums, die einen über den Normalbereich erhöhten Wert an Fetten, insbesondere Triglyzeriden, aufweist. Die Normalbereichsgrenze wird üblicherweise bei 150 mg/dL, teilweise auch bei 200 mg/dL Triglyzeride angegeben.

Der Begriff "EDTA-Plasma oder Serum" umfasst eine Plasmaprobe eines Individuums, bevorzugt eine Plasmaprobe eines menschlichen Individuums, die mit EDTA antikoaguliert wurde (EDTA-Plasma) oder gar nicht antikoaguliert und spontan geronnen ist (Serum). Zur Gewinnung des Plasma- oder Serum-Überstands wird die Blutprobe zentrifugiert.

Der Begriff "Plasma oder Serum mit Bilirubin" umfasst eine Plasma- oder Serumprobe eines Individuums, bevorzugt eine Plasma- oder Serumprobe eines menschlichen Individuums, die einen Bilirubingehalt oberhalb des Normalbereichs aufweist. Die Obergrenze des Normalbereichs wird häufig mit 1,2 mg/dL angegeben.

Der Begriff "Plasma oder Serum mit Hämoglobin" umfasst eine Plasma- oder Serumprobe eines Individuums, bevorzugt eine Plasma- oder Serumprobe eines menschlichen Individuums, die einen Hämoglobingehalt oberhalb des Normalbereichs aufweist. Dessen Obergrenze wird bei Plasma häufig mit 20 mg/dL und bei Serum mit 50 mg/dL angegeben.

Der Begriff "Serum" umfasst eine Serumprobe eines Individuums, bevorzugt eine Serumprobe eines menschlichen Individuums, und bezeichnet den zellfreien Überstand des Blutes nach Abschluss der Gerinnung und vorteilhafterweise nach Zentrifugation.

Unter dem Begriff "Absorption" ist im allgemeineren Sinn auch "Extinktion" zu verstehen. Insbesondere ist unter einer "Messung einer Absorption" ist im allgemeineren Sinn auch die "Messung einer Extinktion" zu verstehen.

Der Begriff "ln(x)" bezeichnet Logarithmen zur Basis e, wobei e die eulersche Zahl bezeichnet, die näherungsweise 2,71828 beträgt. Logarithmen zur Basis e werden allgemein auch als natürliche Logarithmen bezeichnet.

Soweit im Kontext dieser Anmeldung Ungleichungen, d.h. z.B. Aussagen oder Bedingungen, dass ein erster Wert kleiner als ein zweiter Wert sei, angegeben sind, kann jeweils auch die Gleichheit der jeweiligen Werte sinngemäß umfasst sein.

**Kurze Beschreibung der Figuren**

Verschiedene Ausführungsformen und Ausgestaltungen der vorliegenden Erfindung werden nun in Bezug auf die beiliegenden Zeichnungen genauer beschrieben.

Fig. 1 zeigt Messwerte der ersten Absorption A1 von Körperflüssigkeitsproben gemäß einer Ausführungsform der Erfindung;

Fig. 2 zeigt Messwerte der ersten Absorption A1 und der zweiten Absorption A2 von Körperflüssigkeitsproben gemäß einer Ausführungsform der Erfindung.

Die beschriebenen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung.

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt. Gleiche Bezugszeichen bezeichnen dabei gleiche oder ähnlich wirkende Komponenten.

Körperflüssigkeitsproben im Sinne der vorliegenden Erfindung können alle Proben biologischen Ursprungs sein, welche flüssige Konsistenz aufweisen und eine Vielzahl von biologisch aktiven Substanzen in verschiedenen Konzentrationen aufweisen. Beispielsweise können Körperflüssigkeitsproben Blutserum, Blutplasma, Blut, Urin, Lymphflüssigkeit, Gallenflüssigkeit oder ähnliche Flüssigkeiten aufweisen.

Photometrische Messwerte im Sinne der vorliegenden Erfindung können Messwerte sein, welche mit photometrischen Messeinrichtungen und zugehörigen Lichtquellen, insbesondere Lasern, Laserdioden, Leuchtdioden oder dergleichen aufgenommen werden können. Messeinrichtungen umfassen beispielsweise CCD-Sensoren, CMOS-Sensoren, Photosensoren oder ähnliche Einrichtungen, welche dazu geeignet sind, die Intensität eines Lichtstrahls wellenlängenabhängig zu erfassen. Mittel photometrischer Messwerte kann die Absorption einer Körperflüssigkeitsprobe bei vorbestimmten Wellenlängen erfolgen.

Lipide im Sinne der vorliegenden Anmeldung können alle im Wesentlichen hydrophoben organischen Verbindungen umfassen, insbesondere im menschlichen oder tierischen Organismus vorkommende Verbindungen. Lipide im Sinne der Erfindung umfassen dabei insbesondere Fette bzw. Triglyceride bzw. Triacylglycerine, welche im menschlichen Körper vorkommen können.

Extinktionskurven und Extinktionswerte im Sinne der vorliegenden Erfindung können dimensionslose Größen sein, welche ein wellenlängenabhängiges Maß für die Opazität von Körperflüssigkeitsproben gegenüber dem Durchgang von Lichtstrahlen im sichtbaren, infraroten und/oder ultravioletten Wellenlängenbereich angeben. Es kann gleichermaßen auch möglich sein, dass Extinktionswerte in Bezug auf eine Einheitsdicke einer Messzelle oder Küvette, in der Körperflüssigkeitsproben während des Durchtritts von Lichtstrahlen zur Erfassung von Intensitätsmesswerten gehalten werden, angegeben werden. In diesem Fall können die Extinktionswerte eine Dimension von [1/cm] aufweisen. In jedem Fall sind die angegebenen Extinktionswerte der nachfolgenden Ausführungsformen nur beispielhafter Natur und von der Messapparatur, der Probenbeschaffenheit und der Probenzusammensetzung abhängig. Extinktionswerte werden im Folgenden jeweils mit Absorptionswerten gleichgesetzt, obwohl es dem Fachmann klar ist, dass bei dieser Betrachtung Diffraktion, Streuung und Reflexion zwar zu den Extinktionswerten beitragen, gegenüber der Absorption jedoch im betrachteten Wellenlängenbereich im Wesentlichen vernachlässigbar sind.

In Körperflüssigkeitsproben können häufig Hämoglobin, Bilirubin und Lipide, insbesondere Triacylglycerine (Triglyceride), enthalten sein. Zur Zuordnung einer Körperflüssigkeitsprobe zu einer Körperflüssigkeitsprobenklasse aus der Gruppe plättchenreiches Plasma oder Serum (P), Citrat-Plasma (PAP), lipämisches Plasma oder Serum (T), EDTA-Plasma (E), Serum (S), Plasma oder Serum mit Bilirubin (B), und Plasma oder Serum mit Hämoglobin (H) kann es wichtig sein, den Lipidanteil zu bestimmen.

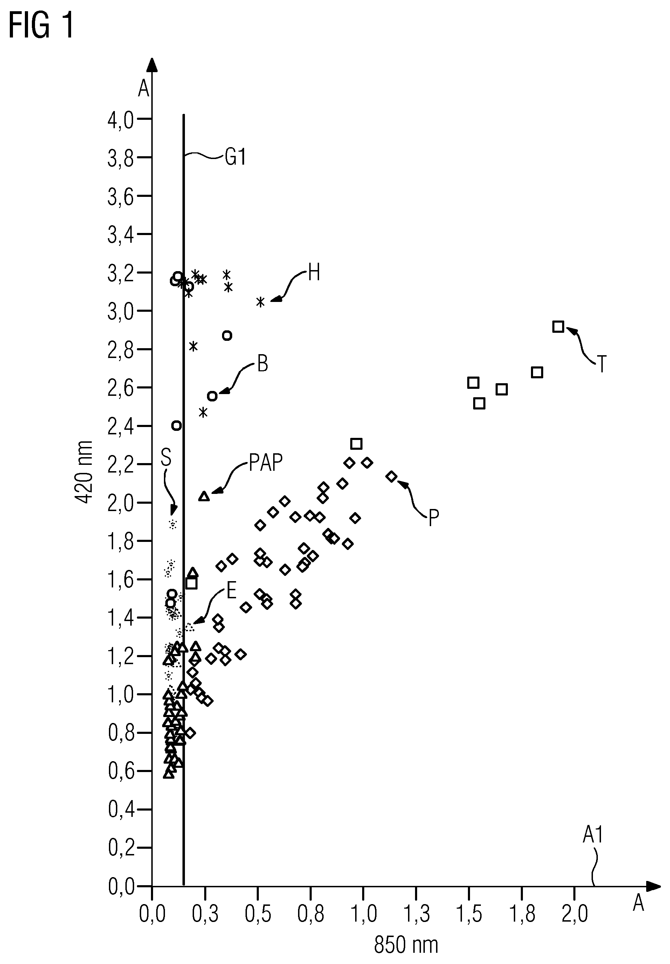

**Fig. 1** zeigt Messwerte der ersten Absorption A1 von Körperflüssigkeitsproben gemäß einer bevorzugten Ausführungsform der Erfindung.

Die erste Wellenlänge beträgt 850 nm und die zweite aufgetragene Wellenlänge 420 nm. Bei der ersten Wellenlänge wird eine erste Absorption A1 der Körperflüssigkeitsprobe gemessen und ein erster Grenzwert G1 der Absorption gebildet. Der erste Grenzwert G1 der Absorption beträgt 0,15 A.

Die Absorption ist in der Einheit A angegeben und bezieht sich auf Messungen an eine Küvette der Schichtdicke 10 mm. Die Einheit A ist dimensionslos dargestellt.

Dargestellt sind exemplarisch Absorptionen von Körperflüssigkeitsproben der Körperflüssigkeitsprobenklassen plättchenreiches Plasma (P), Citrat-Plasma (PAP), lipämisches Plasma(T), Plasma mit Bilirubin (B), Plasma mit Hämoglobin (H), Serum (S) und EDTA-Plasma (E). Nur bei Proben mit einem Absorptionswert oberhalb des ersten Grenzwertes G1 der Absorption kann es sich um Körperflüssigkeitsproben der Körperflüssigkeitsprobenklasse plättchenreiches Plasma oder Serum (P) handeln.

Die Anzahl der gemessenen Proben betrug 47 für plättchenreiches Plasma (P), 50 für Citrat-Plasma (PAP), 14 für lipämisches Plasma (T), 9 für Plasma mit Bilirubin (B), und 10 für Plasma mit Hämoglobin (H), 20 für Serum (S) und 10 für EDTA-Plasma (E).

Citrat-Plasma (PAP), Plasma oder Serum mit Bilirubin (B) oder Hämoglobin (H), Serum (S), EDTA-Plasma (E), lipämisches Plasma oder Serum(T) bilden eine Körperflüssigkeitsprobenklasse und plättchenreiches Plasma (P) bildet eine Körperflüssigkeitsprobenklasse.

Plättchenreiches Plasma (P) wurde aus dem Überstand nach Zentrifugation von Blut bei 170 g für 15 Minuten oder 150 g oder für 15 Minuten oder bei 180 g für 10 Minuten gewonnen.

(Citrat-) Plasma (PAP) wurde durch Zentrifugation bei 1500 g für 15 Minuten oder bei 1500 g für 10 Minuten oder bei 2000 g für 20 Minuten gewonnen.

Lipämisches Plasma (T) enthält mehr als 150 mg/dL Triglyzeride.

Plasma oder Serum mit Bilirubin (B) enthält mehr als 1,2 mg/dL Billirubin.

Plasma mit Hämoglobin (H) enthält mehr als 20 mg/dL Hämoglobin.

Serum mit Hämoglobin (H) enthält mehr als 50 mg/dL Hämoglobin.

Serum (S) enthält weniger als 1,2 mg/dL Billirubin und/oder weniger als 50 mg/dL Hämoglobin. EDTA-Plasma (E) enthält weniger als 1,2 mg/dL Billirubin und/oder weniger als 400 mg/dL Hämoglobin.

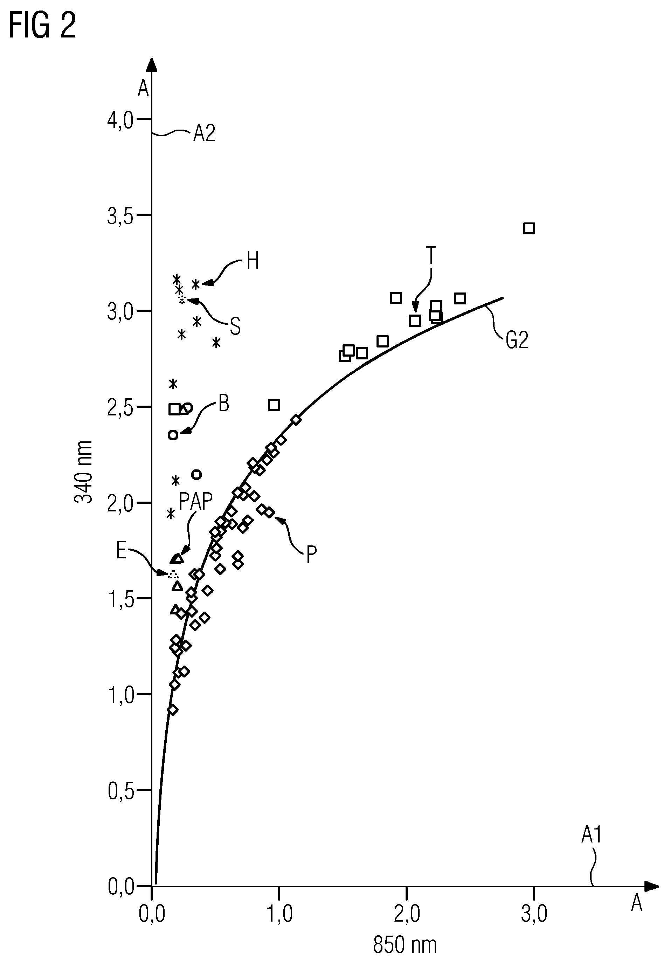

Fig. 2 zeigt Messwerte der ersten Absorption A1 und der zweiten Absorption A2 von Körperflüssigkeitsproben gemäß einer Ausführungsform der Erfindung.

Dargestellt sind Messwerte der Proben aus FIG 1, deren erste Absorption A1 einen Absorptionswert oberhalb des ersten Grenzwertes G1 der Absorption aufweist.

Die erste Wellenlänge beträgt 850 nm und die zweite Wellenlänge 340 nm. Bei der ersten Wellenlänge wird eine erste Absorption A1 der Körperflüssigkeitsprobe und bei der zweiten Wellenlänge wird eine zweite Absorption A2 gemessen.

Ein von der ersten Absorption A1 abhängiger zweiter Grenzwert G2 der Absorption wird gebildet und die zweite Absorption A2 mit dem zweiten Grenzwert G2 der Absorption verglichen. Der zweite Grenzwert der Absorption beträgt $0,7189 \ln(A1) + 2,3413$ A.

Die Körperflüssigkeitsproben werden zu der Körperflüssigkeitsprobenklasse plättchenreiches Plasma (P) zugeordnet, falls A1 größer G1 und A2 kleiner G2, oder der Körperflüssigkeitsprobenklasse Citrat-Plasma (PAP), Plasma oder Serum mit Bilirubin (B), Plasma oder Serum mit Hämoglobin (H), Serum (S), EDTA-Plasma (E), lipämisches Plasma oder Serum (T) zugeordnet, falls A1 kleiner G1 und/oder A2 größer G2.

Die Zuordnung der Körperflüssigkeitsprobe zu einer Körperflüssigkeitsprobenklasse erfolgt automatisch mittels eines Systems mit einer Messeinrichtung, welche dazu ausgelegt ist, eine Körperflüssigkeitsprobe mit Lichtstrahlen mit einer Mehrzahl von Wellenlängen zu durchstrahlen und Messung einer Mehrzahl von Absorptionen der Körperflüssigkeitsprobe bei der Mehrzahl von Wellenlängen durchzuführen. Weiter umfasst das System eine Berechnungseinrichtung für die Berechnung der entsprechenden Schritte des erfindungsgemäßen Verfahrens.

**Bezugszeichenliste**

| | |
|---|---|
| PAP | (Citrat-) Plasma |
| P | plättchenreiches Plasma |
| T | lipämisches Plasma |
| H | Plasma mit Hämoglobin |
| B | Plasma mit Bilirubin |
| S | Serum |
| E | EDTA-Plasma |
| A1 | erste Absorption |
| A2 | zweite Absorption |
| G1 | erster Grenzwert der Absorption |
| G2 | zweiter Grenzwert der Absorption |

**Patentansprüche**

1. Verfahren zur Zuordnung einer Körperflüssigkeitsprobe aus der Gruppe Citrat-Plasma (PAP), Plasma mit Bilirubin (B), Serum mit Bilirubin, Plasma mit Hämoglobin (H), Serum mit Hämoglobin, lipämisches Plasma oder Serum (T), EDTA-Plasma (E), Serum (S), und plättchenreiches Plasma (P) zu einer Körperflüssigkeitsprobenklasse mit den Schritten:

   a) Durchstrahlen der Körperflüssigkeitsprobe mit Licht bei zwei Wellenlängen und
   b) Messung der Absorptionen der Körperflüssigkeitsprobe bei den zwei Wellenlängen,

   **gekennzeichnet durch** die Schritte:

   c) Messung einer ersten Absorption A1 der Körperflüssigkeitsprobe bei einer ersten Wellenlänge, die im Bereich zwischen 610 nm und 930 nm liegt,
   d) Messung einer zweiten Absorption A2 der Körperflüssigkeitsprobe bei einer zweiten Wellenlänge, die im Bereich zwischen 320 nm und 420 nm liegt,
   e) Vergleich der ersten Absorption A1 mit einem vorbestimmten ersten Grenzwert G1 der Absorption,
   f) Bildung eines von der ersten Absorption A1 abhängigen zweiten Grenzwerts G2 der Absorption und Vergleich der zweiten Absorption A2 mit dem zweiten Grenzwert G2 der Absorption,
   g) Zuordnung der Körperflüssigkeitsprobe zu der Körperflüssigkeitsprobenklasse plättchenreiches Plasma (P), falls A1 größer G1 und A2 kleiner G2, oder
   h) Zuordnung der Körperflüssigkeitsprobe zu der Körperflüssigkeitsprobenklasse Citrat-Plasma (PAP), Plasma mit Bilirubin (B), Serum mit Bilirubin, Plasma mit Hämoglobin (H), Serum mit Hämoglobin, lipämisches Plasma oder Serum (T), Serum (S), oder EDTA-Plasma (E), falls A1 kleiner G1 oder A2 größer G2,

   wobei Citrat-Plasma (PAP), Plasma mit Bilirubin (B), Serum mit Bilirubin, Plasma mit Hämoglobin (H), Serum mit Hämoglobin, lipämisches Plasma oder Serum (T), Serum (S), oder EDTA-Plasma (E) eine Körperflüssigkeitsprobenklasse bildet und
   wobei plättchenreiches Plasma (P) eine Körperflüssigkeitsprobenklasse bildet.

2. Verfahren nach Anspruch 1, wobei die erste Wellenlänge 850 nm und/oder die zweite Wellenlänge 340 nm beträgt.

3. Verfahren nach Anspruch 1, wobei die erste Wellenlänge 645 nm und/oder die zweite Wellenlänge 365 nm beträgt

4. Verfahren nach Anspruch 1, wobei die erste Wellenlänge 800 oder 660 nm und/oder die zweite Wellenlänge 340 oder 405 nm beträgt

5. Verfahren nach einem der vorhergehenden Ansprüche, wobei der erste Grenzwert der Absorption G1 0,15 A und/oder der zweite Grenzwert der Absorption G2 $0{,}7189 \ln(A1) + 2{,}3413$ A beträgt.

6. Verfahren nach einem der Ansprüche 1 bis 5, wobei das Durchstrahlen der Körperflüssigkeitsprobe mithilfe von Laser- oder Leuchtdioden und das Erfassen der Mehrzahl von Absorptionen (A1; A2) mithilfe eines photometrischen Sensors erfolgt.

7. System zur Zuordnung einer Körperflüssigkeitsprobe aus der Gruppe Citrat-Plasma (PAP), Plasma mit Bilirubin (B), Serum mit Bilirubin, Plasma mit Hämoglobin (H), Serum mit Hämoglobin, lipämisches Plasma oder Serum (T) oder EDTA-Plasma (E), Serum (S), und plättchenreiches Plasma (P) zu einer Körperflüssigkeitsprobenklasse, mit:

   I) einer Messeinrichtung, welche dazu ausgelegt ist, eine Körperflüssigkeitsprobe mit Lichtstrahlen mit zwei Wellenlängen zu durchstrahlen und Messung von zwei Absorptionen der Körperflüssigkeitsprobe bei den zwei Wellenlängen durchzuführen, wobei die Messung einer ersten Absorption A1 der Körperflüssigkeitsprobe bei einer ersten Wellenlänge erfolgt, die im Bereich zwischen 610 nm und 930 nm liegt, und die Messung einer zweiten Absorption A2 der Körperflüssigkeitsprobe bei einer zweiten Wellenlänge erfolgt, die im Bereich zwischen 320 nm und 420 nm liegt, und
   II) einer Berechnungseinrichtung, welche dazu ausgelegt ist, die erste Absorption A1 mit einem vorbestimmten ersten Grenzwert G1 der Absorption zu vergleichen, und
   III) einer Berechnungseinrichtung, welche dazu ausgelegt ist, einen von der ersten Absorption A1 abhängigen zweiten Grenzwert G2 der Absorption zu bilden und die zweite Absorption A2 mit dem zweiten Grenzwert G2 der Absorption zu vergleichen, und

IV) einer Berechnungseinrichtung, welche dazu ausgelegt ist, die Körperflüssigkeitsprobe zu der Körperflüssigkeitsprobenklasse plättchenreiches Plasma (P) zuzuordnen, falls A1 größer G1 und A2 kleiner G2 ist, oder

die Körperflüssigkeitsprobe zu der Körperflüssigkeitsprobenklasse Citrat-Plasma (PAP), Plasma mit Bilirubin (B), Serum mit Bilirubin, Plasma mit Hämoglobin (H), Serum mit Hämoglobin, lipämisches Plasma oder Serum (T), Serum (S) oder EDTA-Plasma (E) zuzuordnen, falls A1 größer G1 oder A2 größer G2 ist, wobei Citrat-Plasma (PAP), Plasma mit Bilirubin (B), Serum mit Bilirubin, Plasma mit Hämoglobin (H), Serum mit Hämoglobin, lipämisches Plasma oder Serum (T), Serum (S), oder EDTA-Plasma (E) eine Körperflüssigkeitsprobenklasse bildet und

wobei plättchenreiches Plasma (P) eine Körperflüssigkeitsprobenklasse bildet.

8. System nach Anspruch 7, wobei die erste Wellenlänge 850 nm und/oder die zweite Wellenlänge 340 nm beträgt.

9. System nach Anspruch 7, wobei die erste Wellenlänge 645 nm und/oder die zweite Wellenlänge 365 nm beträgt

10. System nach Anspruch 7, wobei die erste Wellenlänge 800 oder 660 nm und/oder die zweite Wellenlänge 340 oder 405 nm beträgt.

11. System nach einem der Ansprüche 7 bis 10, wobei der erste Grenzwert der Absorption G1 0,15 A und/oder der zweite Grenzwert der Absorption G2 0,7189 ln(A1) + 2,3413 A beträgt.

12. System nach einem der Ansprüche 7 bis 11, wobei das Durchstrahlen der Körperflüssigkeitsprobe mithilfe von Laser- oder Leuchtdioden und das Erfassen der Mehrzahl von Absorptionen (A1; A2) mithilfe eines photometrischen Sensors erfolgt.

13. Automatischer Analysator umfassend ein System nach einem der Ansprüche 7 bis 12.

14. Verwendung eines Systems nach einem der Ansprüche 7 bis 12 in einem automatischen Analysator.

FIG 1

## FIG 2

Europäisches
Patentamt

European
Patent Office

Office européen
des brevets

# EUROPÄISCHER RECHERCHENBERICHT

Nummer der Anmeldung

EP 16 18 8802

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2015/073886 A1 (TERUMOBCT INC [US]) 21. Mai 2015 (2015-05-21) * Zusammenfassung; Abbildung 4 * * Absätze [0063], [0073] - [0074] * ----- | 1-14 | INV. G01N33/49 G01N21/31 |
| A | US 4 810 090 A (BOUCHER TERRY D [US] ET AL) 7. März 1989 (1989-03-07) * Zusammenfassung; Abbildungen 1,2,4 * * Spalte 3, Zeile 66 - Spalte 6, Zeile 15 * ----- | 1-14 | |
| A | EP 3 051 271 A1 (SIEMENS HEALTHCARE DIAGNOSTICS [DE]) 3. August 2016 (2016-08-03) * Zusammenfassung; Abbildung 1 * * Absätze [0020] - [0023] * ----- | 1-14 | |
| A,D | EP 2 549 264 A1 (SIEMENS HEALTHCARE DIAGNOSTICS [DE]) 23. Januar 2013 (2013-01-23) * Zusammenfassung; Abbildung 2 * * Absätze [0012] - [0020] * ----- | 1-14 | |

| | | | RECHERCHIERTE SACHGEBIETE (IPC) |
|---|---|---|---|
| A | US 2004/079707 A1 (SMITH KELLY B [US] ET AL) 29. April 2004 (2004-04-29) * Zusammenfassung * * Absätze [0113] - [0115] * ----- | 1-14 | G01N G01J |
| A | US 2001/024800 A1 (GARCIA-RUBIO LUIS HUMBERTO [US] ET AL) 27. September 2001 (2001-09-27) * Absätze [0017], [0025] - [0027] * ----- | 1-14 | |

Der vorliegende Recherchenbericht wurde für alle Patentansprüche erstellt

| Recherchenort | Abschlußdatum der Recherche | Prüfer |
|---|---|---|
| München | 23. März 2017 | Meacher, David |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 16 18 8802

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-03-2017

| Im Recherchenbericht angeführtes Patentdokument | Datum der Veröffentlichung | Mitglied(er) der Patentfamilie | Datum der Veröffentlichung |
|---|---|---|---|
| WO 2015073886 A1 | 21-05-2015 | EP 3068542 A1<br>US 2015140546 A1<br>WO 2015073886 A1 | 21-09-2016<br>21-05-2015<br>21-05-2015 |
| US 4810090 A | 07-03-1989 | AU 610029 B2<br>AU 2108488 A<br>CA 1325162 C<br>DE 3828618 A1<br>FR 2619923 A1<br>GB 2208927 A<br>IT 1223785 B<br>JP H0197437 A<br>JP H0369532 B2<br>US 4810090 A | 09-05-1991<br>02-03-1989<br>14-12-1993<br>16-03-1989<br>03-03-1989<br>19-04-1989<br>29-09-1990<br>14-04-1989<br>01-11-1991<br>07-03-1989 |
| EP 3051271 A1 | 03-08-2016 | CN 105823739 A<br>EP 3051271 A1<br>EP 3051272 A2<br>JP 2016138886 A<br>US 2016216249 A1 | 03-08-2016<br>03-08-2016<br>03-08-2016<br>04-08-2016<br>28-07-2016 |
| EP 2549264 A1 | 23-01-2013 | CN 103649721 A<br>EP 2549264 A1<br>EP 2710348 A1<br>JP 2014521095 A<br>US 2014192342 A1<br>WO 2013010970 A1 | 19-03-2014<br>23-01-2013<br>26-03-2014<br>25-08-2014<br>10-07-2014<br>24-01-2013 |
| US 2004079707 A1 | 29-04-2004 | KEINE | |
| US 2001024800 A1 | 27-09-2001 | US 7691642 B1<br>US 2001024800 A1 | 06-04-2010<br>27-09-2001 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2549264 A1